# EUROPÄISCHE PATENTSCHRIFT

(11) **EP 3 256 174 B1**
(45) Veröffentlichungstag und Bekanntmachung des Hinweises auf die Patenterteilung: **08.10.2025**
(21) Anmeldenummer: 16703762.1
(22) Anmeldetag: 08.02.2016
(51) Int. Cl.: A61L 2/28

(54) **STERILISATOR**
STERILISER
STÉRILISATEUR

(30) Priorität: 12.02.2015 EP 15154796
(43) Veröffentlichungstag der Anmeldung: 20.12.2017
(73) Patentinhaber: Belimed AG, 6300 Zug (CH)
(72) Erfinder: STADLER, Hans, 8583 Sulgen (CH); DÜRING, Daniel, 9246 Niederbüren (CH)
(74) Vertreter: Petraz, Davide Luigi
(86) Internationale Anmeldenummer: PCT/EP2016/052623
(87) Internationale Veröffentlichungsnummer: WO 2016/128351

(56) Entgegenhaltungen:
- EP-A1- 0 982 039
- EP-A1- 1 342 478
- EP-A1- 2 366 411
- EP-A2- 1 844 838
- WO-A1-00/06211
- WO-A2-2012/135694
- US-A- 3 245 461
- US-A1- 2007 274 858

## Beschreibung

Die vorliegende Erfindung betrifft einen Sterilisator nach dem Oberbegriff von Anspruch 1.

Sterilisatoren, insbesondere Dampfsterilisatoren, werden im klinischen Umfeld und in verwandten Bereichen verwendet, um die weitgehende Keimfreiheit von Sterilgut zu gewährleisten. Bei Sterilgut handelt es sich in der Regel um medizinische Geräte oder um Arbeitskleidung von unter sterilen Bedingungen arbeitendem Personal.

Um die erforderliche Keimfreiheit im Routinebetrieb zu gewährleisten, muss die Effektivität eines Sterilisationsprozesses in regelmässigen Zeitintervallen überprüft werden. Diese Überprüfung erfolgt normalerweise indirekt mittels einer Prüfeinrichtung, welche den Sterilisationsbedingungen ausgesetzt wird.

Im medizinischen Bereich sind Dampfsterilisatoren mit Vorvakuum weit verbreitet. Bei solchen Sterilisatoren wird die Sterilisation in einer Sterilisierkammer durchgeführt. Das dabei angewendete Verfahren beinhaltet im Wesentlichen drei Phasen. In einer ersten Phase, der so genannten Entlüftungsphase, auch Vorbehandlungsphase genannt, wird die Sterilisierkammer evakuiert und die darin enthaltene Luft durch Dampf ersetzt. Dieser Prozess kann mehrfach wiederholt werden. Man spricht dann auch von einem sog. fraktionierten Vorvakuum. In der zweiten Phase erfolgt die eigentliche Sterilisation, wobei der Dampf unter vorgegebenem Druck und Temperatur für eine bestimmte Zeit auf das Sterilgut in der Sterilisierkammer einwirkt. Die dritte Phase beinhaltet eine Trocknung, bei welcher Kondensat im Innenraum der Sterilisierkammer durch Vakuum und Erwärmen entfernt wird.

Bei medizinischen Dampfsterilisatoren mit fraktioniertem Vorvakuum ist die routinemässige Leistungsüberprüfung mit dem sogenannten Bowie-Dick-Test vorgeschrieben. Dieser Test simuliert die schwierige Dampfdurchdringung eines festgepressten Paketes aus 7 kg Textilien. Nach der Norm ISO 11140-4 für verpacktes Sterilgut und poröse Ladungen ist ein solcher Nachweis obligatorisch. Er dient weiterhin dem Nachweis der Konformität mit der Norm EN 285 und sollte als Routineprüfung nach ISO 17665-1 einmal täglich durchgeführt werden, um die Funktion des Vorvakuums zu überprüfen. Eine mögliche Testanordnung für den Bowie-Dick-Test umfasst einen Stapel von fest zusammengepresstem saugfähigem Papier, in welchen eine Testkarte eingelegt ist. Auf der Testkarte sind chemische Indikatoren aufgebracht, welche die Sterilisationswirkung durch eine Farbveränderung anzeigen.

Allerdings können chemische Indikatoren auch in einen gasdurchlässigen Testbehälter eingelegt und den Sterilisationsbedingungen ausgesetzt werden. Wenn vor dem Einleiten des Dampfes die Luft im Sterilisator nicht ausreichend entfernt wird, erreicht die Dampfsättigung im Testbehälter nicht die notwendige Konzentration, wodurch die mangelhafte Funktion des Sterilisators durch die Indikatoren sichtbar wird. Neben chemischen Indikatoren können beim Bowie-Dick-Test auch elektronische Sensoren eingesetzt werden. So kann beispielsweise ein Prüfkörper bestehend aus einem System von Hohlräumen mit einem oder mehreren Temperatursensoren den Sterilisationsbedingungen ausgesetzt werden. Durch eine oder mehrere Temperaturmessungen am Prüfkörper kann entschieden werden, ob ein Sterilisationsprozess erfolgreich war.

Die DE 10 2010 016 017 A1 sowie die EP 2 366 411 A1 beschreiben einen Sterilisator mit einer Sterilisierkammer und einer Prüfeinrichtung zur Prüfung der Effektivität des Sterilisationsprozesses. Die besagte Prüfeinrichtung umfasst einen Prüfkörper und eine Sonde, wobei die Sonde im Prüfkörper angebracht ist. Die Prüfeinrichtung ist dabei derart ausgelegt, dass der Prüfkörper ausserhalb und die Sonde mindestens teilweise innerhalb der Sterilisierkammer angeordnet ist. Ferner ist die Prüfeinrichtung im Regelfall mit der Sterilisierkammer fest verbunden. Eine derartige Vorrichtung hat allerdings den Nachteil, dass sich im Prüfkörper ansammelndes Kondensat nur sehr langsam über Verdampfung entfernen lässt. Zudem ist die beschriebene Konstruktionsweise durch das Vorhandensein von Komponenten ausserhalb der Sterilisierkammer vergleichsweise sperrig.

Die WO 00/06211 A1 beschreibt ein Verfahren zur Echtzeit-Überwachung der Effektivität eines Sterilisationsprozesses in einem entsprechend ausgerüsteten Sterilisator. Der Sterilisator verfügt hierzu über eine Sonde, welche über eine Öffnung mit dem Innenraum einer Sterilisierkammer in Verbindung bringbar ist. Ausserhalb der Sterilisierkammer ist eine Sensoreinheit angebracht, an welcher eine Reihe von Sensoren angeordnet sind und mit der Sonde in Verbindung stehen.

Es ist die Aufgabe der Erfindung, die Nachteile im Stand der Technik zu überwinden.

Insbesondere ist es eine Aufgabe der vorliegenden Erfindung, einen Sterilisator mit einer Sterilisierkammer und mit einer Prüfeinrichtung zur Prüfung der Effektivität eines Sterilisationsprozesses zu schaffen, welcher zuverlässig im Betrieb und einfach in der Handhabung ist. Der Sterilisator soll zudem vielfältig anwendbar und konstruktiv einfach ausgestaltet sein. Er soll eine kompakte Bauweise aufweisen und kostengünstig herstellbar sein. Ferner soll ein wartungsarmer Betrieb möglich sein.

Diese Aufgaben werden durch einen Sterilisator gelöst, welcher die Merkmale in Anspruch 1 aufweist.

Die Erfindung betrifft einen Sterilisator mit einer Sterilisierkammer und mit einer Prüfeinrichtung zur Prüfung der Effektivität eines Sterilisationsprozesses. Eine solche Prüfung kann das Testen der Effektivität einer erfolgreichen Entlüftung während des Sterilisationsprozesses beinhalten, wie es in der Norm EN 285 für einen Bowie-Dick-Test vorgeschrieben ist. Die Prüfeinrichtung umfasst einen Prüfkörper mit einem Sensor zum Messen von zumindest einem Parameter und eine Kühlung zum Kühlen des Prüfkörpers. Die Erfindung ist dadurch gekennzeichnet, dass die gesamte Prüfeinrichtung vollständig im Innenraum der Sterilisierkammer angeordnet ist.

Unter dem Begriff "Kühlung zum Kühlen des Prüfkörpers" sind im vorliegenden Zusammenhang Mittel zu verstehen, mit denen ein Wärmeübergang vom Prüfkörper auf ein anderes Medium erzielt werden kann.

Durch diese Anordnung der Prüfeinrichtung ist es ohne weitere konstruktive Massnahmen möglich, die Prüfeinrichtung in der dritten Phase des Sterilisationsprozesses kontrolliert unter Vakuum zu erwärmen, wodurch angefallenes Kondensat leichter verdampft werden kann. Unter Kondensat wird in diesem Zusammenhang Sterilisationsagens Verstanden, das während dem Sterilisationsprozess in flüssiger Form innerhalb der Sterilisierkammer angefallen ist. Bei klassischen Dampfsterilisatoren handelt es sich dabei typischerweise um kondensierten Wasserdampf.

Darüber hinaus führt das Anordnen der Prüfeinrichtung im Inneren der Prüfkammer zu einer kompakteren Bauweise des gesamten Sterilisators. Auch ist durch das direkte Vorhandensein der Prüfeinrichtung im Innenraum der Sterilisierkammer eine sehr direkte Messung der Effektivität des Sterilisationsprozesses möglich. Die Auswertung der Prüfresultate kann effizient und mit vorteilhaftem Komfort erfolgen, da die Prüfeinrichtung fest in der Sterilisierkammer angeordnet ist und zu diesem Zweck nicht entfernt werden muss.

Die Prüfeinrichtung kann zusätzlich eine Sonde umfassen, die an den Prüfkörper angeschlossen ist. Diese Sonde kann als Hohlkörper zur Leitung von Sterilisationsagens, insbesondere von Wasserdampf und/oder anderen Gasen, ausgebildet sein. Dieser Hohlkörper kann als eine aus Kunststoff und/oder Metall bestehende Leitung ausgebildet und am dem Prüfkörper abgewandten Ende offen sein, um Sterilisationsagens und/oder Wärme zum Prüfkörper zu leiten. Eine derartige Sonde kann, beispielsweise durch mehrfaches Aufrollen, derart ausgestaltet sein, dass die Prüfeinrichtung zur Prüfung der Effektivität des Sterilisationsprozesses von medizinischen Geräten geeignet ist, welche aufgrund ihrer Geometrie oder sonstigen Eigenschaften besondere Anforderung an den Sterilisationsprozess stellen.

Der Prüfkörper kann als, vorzugsweise zylindrische, Kapsel mit einem Innenraum ausgeführt sein. Dies stellt eine bevorzugte Geometrie des Prüfkörpers dar, die leicht zu fertigen ist und besonders günstige Prüfeigenschaften aufweist.

Der Innenraum der Kapsel kann direkt oder indirekt über die Sonde mit der Sterilisierkammer in Fluidkommunikation stehen. Bei einer solchen Ausführung wird in der ersten Phase des Sterilisationsprozesses die sich im Prüfkörper befindliche Luft durch das angelegte Vorvakuum über die Sonde mit dem Sterilisationsagens (beispielsweise Wasserdampf) ausgetauscht. Dadurch kommt es, je nach Sterilisationsagens, insbesondere zu einer Erwärmung des gekühlten Prüfkörpers. Wäre die Effektivität des Sterilisationsprozesses durch das Vorhandensein von Restluft, beispielsweise wegen einer Leckage, innerhalb der Sterilisierkammer herabgesetzt, wäre innerhalb des Prüfkörpers eine Temperaturabweichung gegenüber einem "idealen" Prozess detektierbar. Diese Temperaturabweichung kann sich unter anderem auch in einer zeitlichen Verzögerung bis zum Erreichen einer gewissen Temperatur innerhalb des Prüfkörpers äussern.

Der Sensor und die Sonde können, insbesondere stirnseitig, an unterschiedlichen Enden der Kapsel angeschlossen oder angeordnet sein. Durch diese Anordnung von Sonde und Sensor kann eine besonders empfindliche Prüfung der Effektivität eines Sterilisationsprozesses erzielt werden.

Der Sensor kann zum Messen eines Parameters ausgewählt sein aus einer Liste bestehend aus Temperatur, Druck und Feuchtigkeit. Diese Grössen lassen einen eindeutigen Rückschluss auf die Effektivität eines Sterilisationsprozesses zu. Zudem ist für diese Parameter eine grosse Anzahl von Sensoren verschiedenster Spezifikationen kommerziell erhältlich.

Die Sterilisierkammer kann in einen Beschickungsbereich und einen Prüfbereich unterteilt sein. Der Prüfbereich kann vorzugsweise unterhalb oder seitlich des Beschickungsbereichs angeordnet sein. Diese Anordnung hat den Vorteil, dass sich der Prüfbereich über die kälteste Region der Sterilisierkammer neben ihrem Ablauf erstreckt. Allerdings ist es in gewissen Fällen auch vorteilhaft, wenn der Beschickungsbereich unterhalb oder seitlich des Prüfbereiches angeordnet ist. Die Prüfeinrichtung ist in beiden Fällen vorzugsweise im Prüfbereich angeordnet.

Die Kühlung zum Kühlen des Prüfkörpers kann einen Wärmeübertragungsabschnitt zum Durchleiten eines Kühlmittels oder eines Kältemittels, insbesondere einen Kühlmantel oder eine Kühlspirale, umfassen. Durch eine solche aktive Kühlung des Prüfkörpers kann eine besonders effiziente Kontrolle seiner Temperatur erzielt werden.

Die Kühlung zum Kühlen des Prüfkörpers kann zusätzlich eine Zuleitung zum Zuleiten des Kühlmittels oder des Kältemittels in die Sterilisierkammer und eine Ableitung zum Ableiten des Kühlmittels oder des Kältemittels aus der Sterilisierkammer umfassen. Allerdings kann an der Kühlung zum Kühlen des Prüfkörpers auch eine Zuleitung zum Zuleiten des Kühlmittels oder des Kältemittels in die Sterilisierkammer und eine Ableitung zum Ableiten des Kühlmittels oder des Kältemittels aus der Sterilisierkammer angeschlossen sein. Diese Ausführungen ermöglichen eine besonders effiziente Wärmeabfuhr aus der Sterilisierkammer.

Das Kühlmittel oder das Kältemittel kann in einem Kreislauf zirkulierbar sein. Dieser Kreislauf kann komplett im Sterilisator angeordnet sein. Dadurch ist es möglich, einen erfindungsgemässen Sterilisator unabhängig von weiterer Infrastruktur wie beispielsweise einer Kühlwasserversorgung zu betreiben.

Bei einem Sterilisator mit einem Kühlmittel kann das Kühlmittel aus einer Reinsubstanz oder einem Stoffgemisch zusammengesetzt sein, wobei zumindest eine Vorläufersubstanz des Kühlmittels ausgewählt ist aus einer Liste bestehend aus Wasser, Ethylenglykol, Methanol, Ethanol, Propanol, Isopropanol, Aceton, Luft und Thermalöl. Mischungen dieser Kühlmittel zeichnen sich unter anderem durch einen besonders niedrigen Schmelzpunkt, gute fluidmatische Eigenschaften und gutes Korrosionsverhalten aus.

Bei einem Sterilisator mit einem Kältemittel kann das Kältemittel aus einer Reinsubstanz oder einem Stoffgemisch zusammengesetzt sein, wobei zumindest eine Vorläufersubstanz des Kältemittels ausgewählt ist aus einer Liste bestehend aus Ammoniak, Kohlenstoffdioxid, Wasser, einem Kohlenwasserstoff, einem HFCKW, einem HFKW, einem FCKW und einem FKW. Dabei handelt es sich um langjährig eingesetzte Kältemittel, die in Kombination mit einer breiten Auswahl von Kältemaschinen einsetzbar sind.

Ein vom Sensor erzeugtes, vorzugsweise elektrisches, Signal kann über eine Kabelverbindung aus der Sterilisierkammer führbar sein. Eine derartige Kabelverbindung stellt eine zuverlässige und kostengünstige Anbindung des Sensors dar. Neben einem elektrischen Signal ist es auch möglich, einen vom Sensor gemessenen Wert durch ein optisches Signal, vorzugsweise über einen optischen Lichtwellenleiter, aus der Sterilisierkammer zu leiten.

Allerdings kann ein vom Sensor erzeugtes, vorzugsweises elektrisches, Signal auch über eine Funkverbindung, umfassend einen Sender und einen Empfänger, aus der Sterilisierkammer führbar sein. Eine Funkverbindung hat den Vorteil, dass keine Leitungen durch eine Wand der Sterilisierkammer geführt werden müssen. Damit entfallen mögliche Dichtigkeitsprobleme. Ausserdem lassen sich Sensoren auf diese Weise einfacher austauschen, was eine variable Instrumentierung der Sterilisierkammer vereinfacht.

Jedoch kann ein vom Sensor erzeugtes, vorzugsweise elektrisches, Signal auch induktiv, insbesondere mittels zweier induktiv gekoppelter Spulen, aus der Sterilisierkammer führbar sein. Unter induktiv wird in diesem Zusammenhang verstanden, dass die Verbindung des Signals vom Inneren der Sterilisierkammer zum Äusseren der Sterilisierkammer über zwei Spulen realisiert wird, die induktiv gekoppelt sind. Eine induktive Verbindung hat den Vorteil, dass sie anders als eine Kabelverbindung keinen Durchbruch durch eine Wand der Sterilisierkammer erfordert, hingegen im Vergleich zu einer Funkverbindung eine geringere Störungsanfälligkeit aufweist.

Alle diese Konfigurationen, bei denen ein vom Sensor erzeugtes Signal über eine Kabelverbindung aus der Sterilisierkammer führbar ist, haben den Vorteil, dass die Auswertung während einem laufenden Sterilisierprozess erfolgen kann, womit eine Zeitersparnis erzielbar ist.

Ferner kann ein vom Sensor erzeugtes, vorzugsweise elektrisches, Signal auch von einem sich in der Sterilisierkammer befindlichen Datenspeicher, beispielsweise einem Datenlogger, aufgezeichnet werden.

Die Prüfeinrichtung kann zumindest teilweise in einen an der Sterilisierkammer angebrachten Stutzen, insbesondere einen Validierungsstutzen, eingesetzt sein. Es versteht sich dabei von selbst, dass auch der Innenraum des Stutzens Teil der Sterilisierkammer ist. Diese Anbringung der Prüfeinrichtung hat den Vorteil, dass bestehende Sterilisatoren leicht erfindungsgemäss nachgerüstet werden können. Dies ist im Hinblick auf bestehende Systeme insbesondere vorteilhaft, da die Prüfeinrichtung nicht nur für den als Leertest durchgeführten Bowie-Dick-Test einsetzbar ist, sondern auch als Luftnachweisgerät oder in Verbindung mit einem Chargenkontrollsystem.

Unter einem Luftnachweisgerät wird im vorliegenden Zusammenhang eine Vorrichtung verstanden, welche bei Voll- oder Teilbeladung eines Dampfsterilisators in der ersten Phase des Sterilisationsprozesses, der so genannten Entlüftungsphase, auch Vorbehandlungsphase genannt, eingesetzt wird, um das korrekte Ersetzten der in der Sterilisierkammer enthaltenen Luft mit Wasserdampf zu überprüfen.

Unter einem Chargenkontrollsystem wird im vorliegenden Zusammenhang ein System verstanden, welches bei Voll- oder Teilbeladung eines Dampfsterilisators in der zweiten Phase des Sterilisationsprozesses, der eigentlichen Sterilisationsphase, physikalische Parameter aufzeichnet, welche Rückschlüsse über die Effektivität des Sterilisationsprozesses erlauben.

Bei einer derartigen Anbringung der Prüfeinrichtung innerhalb der Sterilisierkammer kann die Sonde aus dem Stutzen hinaus in einen Prüfbereich innerhalb der Sterilisierkammer hineinragen. Dies ermöglicht, dass die Prüfung dennoch im kältesten Bereich der Sterilisierkammer erfolgen kann.

Die Prüfeinrichtung kann über eine den Stutzen verschliessende Deckelplatte lagestabil im Stutzen und damit in der Sterilisierkammer gehalten sein. Dadurch kann eine denkbar einfache Montierung der Prüfeinrichtung innerhalb der Sterilisierkammer erzielt werden.

Die Deckelplatte kann über einen Schnellverschluss am Stutzen anbringbar sein. Beim besagten Schnellverschluss handelt es bevorzugterweise um eine auf einen den Stutzen abschliessenden Flansch und auf die Deckelplatte wirkende Flanschklammer. Allerdings wären auch andere Varianten eines Schnellverschlusses, wie beispielsweise ein Bajonettverschluss, ein Schraubverschluss oder ein Hebelverschluss, denkbar. Durch die Verwendung eines Schnellverschlusses kann das Einsetzen der Prüfeinrichtung in die Sterilisierkammer besonders zeiteffizient und benutzerfreundlich erfolgen, was insbesondere Wartungsarbeiten oder Validierungsmessungen am Sterilisator deutlich erleichtert.

Weitere Vorteile und Einzelmerkmale der Erfindung ergeben sich aus der nachfolgenden Beschreibung eines Ausführungsbeispiels und aus den Zeichnungen.

Es zeigen schematisch:
- Figur 1:: Darstellung der Sterilisierkammer eines erfindungsgemässen Sterilisators;
- Figur 2:: Zeitlicher Verlauf der durch eine Prüfeinrichtung eines erfindungsgemässen Sterilisators gemessenen Temperatur und des absoluten Druckes innerhalb der Sterilisierkammer über einen typischen Sterilisationsprozess;
- Figur 3:: Teilvergrösserung des durch einen Kreis markierten Bereiches in Figur 2;
- Figur 4:: Perspektivische Darstellung einer Prüfeinrichtung für einen erfindungsgemässen Sterilisator;
- Figur 5:: Teilvergrösserung der Darstellung gemäss Figur 4 als Schnittansicht;
- Figur 6:: Perspektivische Darstellung einer Prüfeinrichtung gemäss den Figuren 4 und 5, eingesetzt in einen Validierungsstutzen einer Sterilisierkammer;
- Figur 7:: Weitere perspektivische Darstellung einer Prüfeinrichtung gemäss den Figuren 4 bis 6, eingesetzt in einen Validierungsstutzen einer Sterilisierkammer.

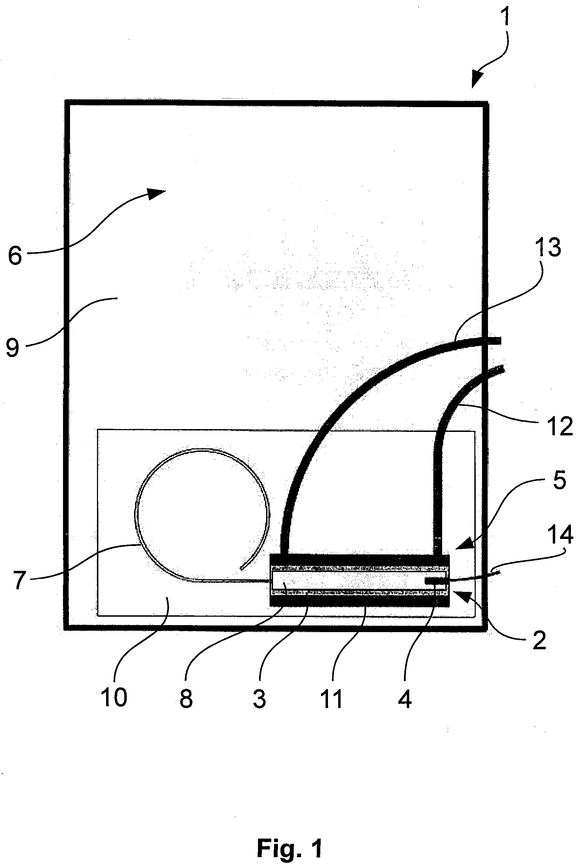

Figur 1 zeigt eine schematische Darstellung der Sterilisierkammer 1 eines erfindungsgemässen Sterilisators. Der Innenraum 6 der Sterilisierkammer 1 ist in einen Beschickungsbereich 9 und einen Prüfbereich 10 unterteilt. Während der Beschickungsbereich 9 zum Beladen mit Sterilisationsgut vorgesehen ist, ist im Prüfbereich 10 eine Prüfeinrichtung 2 zur Prüfung der Effektivität eines Sterilisationsprozesses angeordnet. Die Prüfeinrichtung 2 umfasst einen Prüfkörper 3 in Form einer zylindrischen Kapsel und eine Sonde 7, welche als länglicher Hohlraum ausgebildet ist. Der Innenraum 8 der Kapsel 3 steht dabei über die Sonde 7 mit der Sterilisierkammer in Fluidkommunikation. Im Innenraum 8 der Kapsel 3 ist ein Sensor 4, im vorliegenden Fall ein Temperatursensor, angeordnet. Der Sensor 4 steht über eine Kabelverbindung 14 mit dem Äusseren der Sterilisierkammer 1 in Verbindung. Die Prüfeinrichtung weist ferner eine Kühlung 5 zum Kühlen des Prüfkörpers 3 auf. Dabei gibt der Prüfkörper 3 über einen Wärmeübertragungsabschnitt 11 Energie in Form von Wärme an die Kühlung 5 ab. Im vorliegenden Fall umfasst die Kühlung 5 einen Kühlmantel, der den kapselförmigen Prüfkörper 3 umhüllt. Der Kühlmantel wird über eine Zuleitung 12 vom Äusseren der Sterilisierkammer 1 mit einem Kühlmittel versorgt. Entsprechend ist auch eine Ableitung 13 zum Ableiten des Kühlmittels aus der Sterilisierkammer vorhanden.

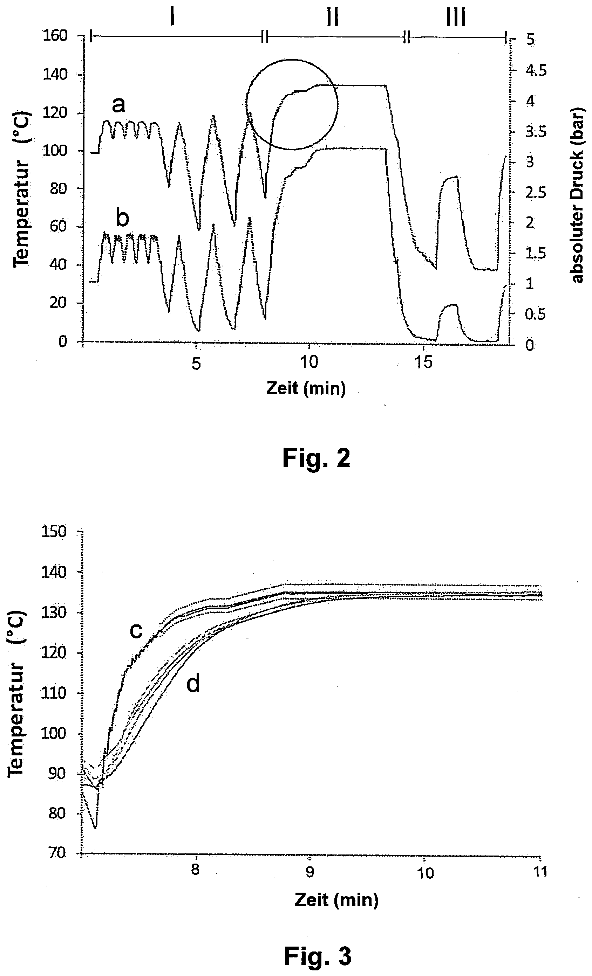

In den Figuren 2 und 3 ist eine Methode zur Prüfung der Effektivität des Sterilisationsprozesses eines erfindungsgemässen Dampfsterilisators beispielhaft erläutert. Im gezeigten Fall arbeitet der Sterilisator unter sogenannten Sattdampfbedingungen. Unter Sattdampf wird in diesem Zusammenhang Wasser verstanden, dessen flüssige und gasförmige Phase gleichzeitig im thermodynamischen Gleichgewicht vorliegen. Unter Sattdampfbedingungen sind Temperatur und Druck voneinander abhängige Grössen, die durch die sogenannte Sattdampfkurve beschrieben werden. Der Verlauf dieser Kurve hängt vom Stoffmengenanteil von Wasser im vorliegenden System ab. Dieser Effekt kann zum Feststellen von verbleibender Luft innerhalb der Sterilisierkammer genützt werden.

Die Kurve a in Figur 2 gibt den Verlauf des absoluten Druckes innerhalb der Sterilisierkammer als Funktion der Zeit über den Sterilisationsprozess wieder. Die Kurve b repräsentiert entsprechend die im Innenraum des Prüfkörpers 8 durch die Sonde 4 gemessene Temperatur. Es ist zu erkennen, dass in einer ersten Phase (I) des Sterilisationsprozesses über eine Reihe verschiedener Zyklen bestehend aus Evakuieren und Befüllen mit Dampf die Luft innerhalb der Sterilisierkammer 1 gänzlich durch Wasserdampf verdrängt wird. Nach dieser ersten Phase erfolgt in einer zweiten Phase (II) der eigentliche Sterilisationsprozess, bei welchem die Sterilisierkammer mit Sattdampf gefüllt und bei einer definierten Temperatur gehalten wird. In der dritten Phase (III) wird erneut mehrfach Vakuum angelegt und der Innenraum der Sterilisierkammer 1 durch gleichzeitiges Erhöhen der Temperatur getrocknet, wodurch angefallenes Kondenswasser entfernt wird. In der besagten dritten Phase (III) kann allerdings auch nur einfach Vakuum angelegt werden.

In Figur 3 ist der durch einen Kreis markierte Bereich in Figur 2 vergrössert dargestellt. Die Kurvenschar c zeigt dabei den typischen Temperaturverlauf unter Sattdampfbedingungen bei einem Stoffmengenanteil an Wasser innerhalb der Sterilisierkammer, welcher den erforderlichen Spezifikationen entspricht. Die Kurvenschar d dagegen zeigt den Temperaturverlauf unter Sattdampfbedingungen mit ungenügendem Stoffmengenanteil an Wasser innerhalb der Sterilisierkammer, beispielsweise durch das Vorhandensein von Restgas. Durch die unterschiedlichen Kurvenverläufe ist deutlich zu erkennen, dass das fraktionierte Vorvakuum bei der Kurvenschar d nicht den Erfordernissen entsprach und der Sterilisationsprozess damit nicht die gewünschte Effektivität hatte.

Bei einem derartigen Sterilisator kann der gemessene Temperaturverlauf automatisch ausgewertet werden, wobei ein laufender Sterilisationsprozess bei Abweichungen der gemessenen Werte von den Spezifikationen abgebrochen wird. Mögliche Bedingungen dafür sind beispielsweise, dass die gemessene Temperatur nicht mehr als 10%, vorzugsweise 5%, bevorzugterweise 2% vom theoretischem Wert abweicht. Eine alternative Bedingung kann gemäss der Norm EN 11140-1 darin bestehen, dass die Temperaturabweichung zu Beginn der Phase (II) (sog. Haltezeit) maximal 1 °C beträgt.

Die gemessenen Temperaturverläufe können zur Qualitätssicherung routinemässig aufgezeichnet und in einer Datenbank, beispielsweise eines sogenannten Chargenkontrollsystems, hinterlegt werden.

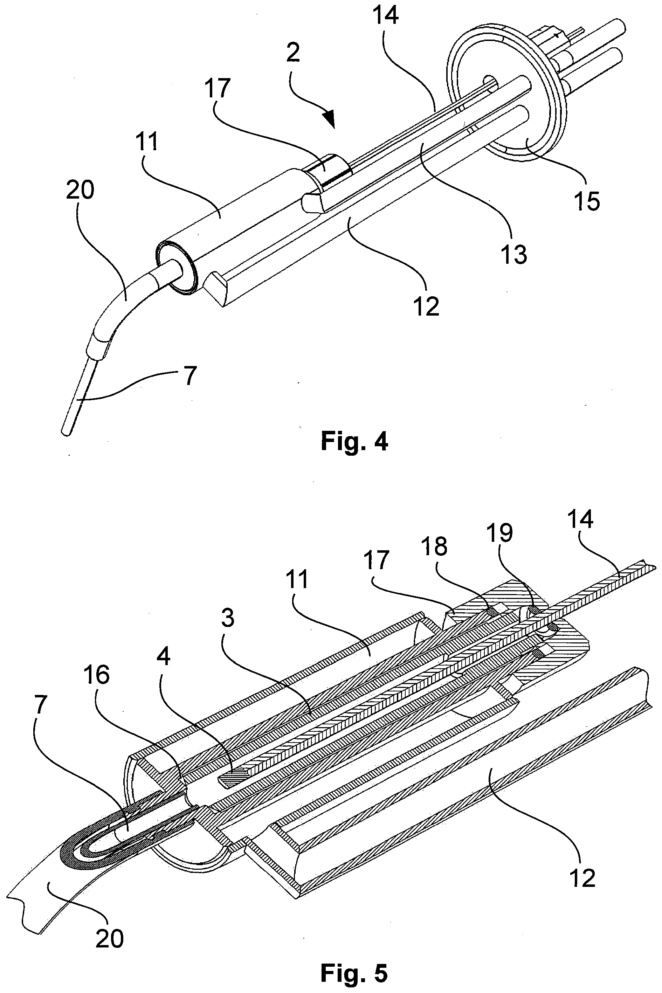

Figur 4 zeigt ein bevorzugtes Ausführungsbeispiel einer Prüfeinrichtung 2 für einen erfindungsgemässen Sterilisator. Beim besagten Beispiel ist der Prüfkörper 3 vollständig vom Wärmeübertragungsabschnitt 11 umgeben, welcher hier als Kühlmantel ausgeführt ist. Die Zufuhr von Kühlmittel erfolgt über die Zuleitung 12, während die Abfuhr desselben über die Ableitung 13 stattfindet. Die Zu- und Ableitungen 12 und 13 sind hier als Edelstahlrohre ausgeführt, welche durch die Deckelplatte 15 geführt sind. Die gezeigte Kühlung ist für das Kühlmittel Luft ausgelegt. Des Weiteren ist ebenfalls eine Kabelverbindung 14 durch die Deckelplatte 15 geführt, welche einen Sensor 4 im Inneren des Prüfkörpers 3 (hier nicht sichtbar) mit dem Äusseren der Sterilisierkammer 1 verbindet. Es ist ersichtlich, dass die Zuleitung 12, die Ableitung 13 und die Kabelverbindung 14 im Wesentlichen parallel geführt sind. An dem der Deckelplatte 15 gegenüberliegenden Ende des Prüfkörpers 3 ist eine rechtwinklig gekrümmte Sonde 7 angebracht.

Aus Figur 5 ergeben sich nähere Einzelheiten zum Prüfkörper 3 und zum Kühlmantel 11. So ist der im Prüfkörper 3 angeordnete Sensor 4, in diesem Fall ein Temperatursensor, erkennbar. Beim Prüfkörper 3 handelt es sich um eine im Wesentlichen zylindrische Kapsel, welche aus einem Keramikmaterial gefertigt ist. Der Kühlmantel 11 bildet eine ebenfalls zylinderförmige Aufnahme 16, in welche der Prüfkörper 3 eingesetzt ist. Die Aufnahme 16 ist mit einem Deckel 17 verschlossen, durch welchen die Kabelverbindung 14 geführt ist. Um einen dichten Verschluss der Aufnahme 16 zu gewährleisten, sind am Deckel 17 Dichtungen 18 und 19 angebracht. An seinem dem Deckel 17 gegenüberliegenden Ende ist der Prüfkörper 3 mit der Sonde 17 verbunden. Die Sonde 17 ist hierzu in Aufnahme 16 eingesteckt und steht stirnseitig mit dem Prüfkörper 3 in Kontakt. Sie Sonde 7 ist durch das Überwurfelement 20 am Kühlmantel 11 gesichert.

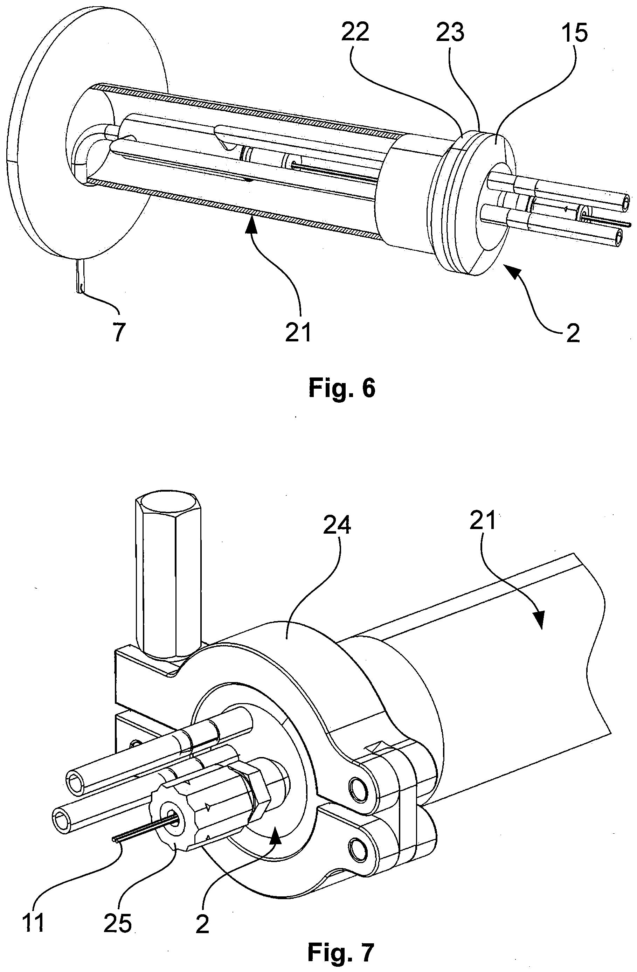

In der Darstellung gemäss Figur 6 ist die vorgängig beschriebene Prüfeinrichtung 2 in einen Validierungsstutzen 21 einer Sterilisierkammer 1 eingesetzt. Dabei ist der Stutzen 21 in Längsrichtung aufgeschnitten gezeigt, so dass die Prüfeinrichtung 2 sichtbar ist. Der Validierungsstutzen 21 weist an seinem der Sterilisierkammer 1 abgewandten Ende einen Flansch 22 auf, auf welchem die Deckelplatte 15 passgenau aufliegt. Zwischen dem Flansch 22 und der Deckelplatte 15 ist ein Dichtelement 23 angebracht. Die Prüfeinrichtung 2 wird lagestabil im Inneren des Validierungsstutzens 21 gehalten, wobei die Sonde 7 in einen Prüfbereich im Inneren der Sterilisierkammer 1 hineinragt.

Figur 7 zeigt ein mit der beschriebenen Prüfeinrichtung 2 versehener Validierungsstutzen 21 aus einer von Figur 6 abweichenden Perspektive. Die Prüfeinrichtung 2 ist am Stutzen 21 über eine Flanschklammer 24 gesichert. Zudem ist ein Deckel 25 gut erkennbar, durch welchen die Kabelverbindung 14 aus der Sterilisierkammer 1 geführt ist.

## Patentansprüche

1. Sterilisator mit einer Sterilisierkammer (1) und mit einer Prüfeinrichtung (2) zur Prüfung der Effektivität eines Sterilisationsprozesses, wobei die Prüfeinrichtung (2) einen Prüfkörper (3) mit einem Sensor (4) zum Messen von zumindest einem Parameter und eine Kühlung (5) zum Kühlen des Prüfkörpers (3) umfasst, **dadurch gekennzeichnet, dass** die gesamte Prüfeinrichtung (2)mit dem Prüfkörper (3) und dem Sensor (4) des Prüfkörpers (3) sowie der Kühlung (5) vollständig im Innenraum (6) der Sterilisierkammer (1) angeordnet ist.

2. Sterilisator nach Anspruch 1, **dadurch gekennzeichnet, dass** die Prüfeinrichtung (2) zusätzlich eine Sonde (7) umfasst, die an den Prüfkörper (3) angeschlossen ist.

3. Sterilisator nach einem der Ansprüche 1 oder 2, **dadurch gekennzeichnet, dass** der Prüfkörper (3) als, vorzugsweise zylindrische, Kapsel mit einem Innenraum (8) ausgeführt ist.

4. Sterilisator nach den Ansprüchen 2 und 3, **dadurch gekennzeichnet, dass** der Innenraum (8) der Kapsel direkt oder indirekt über die Sonde (7) mit der Sterilisierkammer (1) in Fluidkommunikation steht.

5. Sterilisator nach Anspruch 4, **dadurch gekennzeichnet, dass** der Sensor (4) und die Sonde (7), insbesondere stirnseitig, an unterschiedlichen Enden der Kapsel angeschlossen oder angeordnet sind.

6. Sterilisator nach einem der Ansprüche 1 bis 5, **dadurch gekennzeichnet, dass** der Sensor (4) geeignet ist zum Messen eines Parameters ausgewählt aus einer Liste bestehend aus Temperatur, Druck und Feuchtigkeit.

7. Sterilisator nach einem der Ansprüche 1 bis 6, **dadurch gekennzeichnet, dass** die Sterilisierkammer (1) in einen Beschickungsbereich (9) und einen Prüfbereich (10) unterteilt ist, wobei der Prüfbereich (10) vorzugsweise unterhalb oder seitlich des Beschickungsbereiches (9) angeordnet ist, und wobei die Prüfeinrichtung (2) im Prüfbereich (10) angeordnet ist.

8. Sterilisator nach einem der Ansprüche 1 bis 7, **dadurch gekennzeichnet, dass** die Kühlung (5) zum Kühlen des Prüfkörpers (3) einen Wärmeübertragungsabschnitt (11) zum Durchleiten eines Kühlmittels oder eines Kältemittels, insbesondere einen Kühlmantel oder eine Kühlspirale, umfasst.

9. Sterilisator nach Anspruch 8, **dadurch gekennzeichnet, dass** die Kühlung (5) zum Kühlen des Prüfkörpers (3) zusätzlich eine Zuleitung (12) zum Zuleiten des Kühlmittels oder des Kältemittels in die Sterilisierkammer (1) und eine Ableitung (13) zum Ableiten des Kühlmittels oder des Kältemittels aus der Sterilisierkammer (1) umfasst.

10. Sterilisator nach Anspruch 8, **dadurch gekennzeichnet, dass** an der Kühlung (5) zum Kühlen des Prüfkörpers (3) eine Zuleitung (12) zum Zuleiten des Kühlmittels oder des Kältemittels in die Sterilisierkammer (1) und eine Ableitung (13) zum Ableiten des Kühlmittels oder des Kältemittels aus der Sterilisierkammer (1) angeschlossen ist.

11. Sterilisator nach einem der Ansprüche 9 oder 10, **dadurch gekennzeichnet, dass** das Kühlmittel oder das Kältemittel in einem Kreislauf zirkulierbar ist.

12. Sterilisator nach einem der Ansprüche 1 bis 11, **dadurch gekennzeichnet, dass** ein vom Sensor (4) erzeugtes, vorzugsweise elektrisches, Signal über eine Kabelverbindung (14) aus der Sterilisierkammer (1) führbar ist.

13. Sterilisator nach einem der Ansprüche 1 bis 12, **dadurch gekennzeichnet, dass** ein vom Sensor (4) erzeugtes, vorzugsweise elektrisches, Signal über eine Funkverbindung, umfassend einen Sender und einen Empfänger, aus der Sterilisierkammer (1) führbar ist.

14. Sterilisator nach einem der Ansprüche 1 bis 13, **dadurch gekennzeichnet, dass** ein vom Sensor (4) erzeugtes, vorzugsweise elektrisches, Signal induktiv, insbesondere mittels zweier induktiv gekoppelter Spulen, aus der Sterilisierkammer (1) führbar ist.

15. Sterilisator nach einem der Ansprüche 1 bis 14, **dadurch gekennzeichnet, dass** die Prüfeinrichtung (2) zumindest teilweise in einen an der Sterilisierkammer (1) angebrachten Stutzen, insbesondere einen Validierungsstutzen (21), eingesetzt ist.

16. Sterilisator nach Anspruch 15, **dadurch gekennzeichnet, dass** die Sonde (7) aus dem Stutzen hinaus in einen Prüfbereich (10) innerhalb der Sterilisierkammer (1) hineinragt.

17. Sterilisator nach einem der Ansprüche 15 oder 16, **dadurch gekennzeichnet, dass** Prüfeinrichtung (2) über eine den Stutzen verschliessende Deckelplatte (15) lagestabil im Stutzen und damit in der Sterilisierkammer (1) gehalten ist.

18. Sterilisator nach Anspruch 17, **dadurch gekennzeichnet, dass** die Deckelplatte (15) über einen Schnellverschluss am Stutzen anbringbar ist, wobei es sich beim Schnellverschluss bevorzugterweise um eine auf einen den Stutzen abschliessenden Flansch (22) und auf die Deckelplatte (15) wirkende Flanschklammer (24) handelt.

## Claims

1. Sterilizer with a sterilization chamber (1) and with a test device (2) for testing the effectiveness of a sterilization process, the test device (2) comprising a test element (3) with a sensor (4) for measuring at least one parameter and a cooling means (5) for cooling the test element (3), **characterized in that** the entire test device (2) with the test element (3) and the sensor (4) of the test element (3) and the cooling means (5) is arranged completely within the interior (6) of the sterilization chamber (1).

2. Sterilizer according to claim 1, **characterized in that** the test device (2) additionally comprises a probe (7) which is attached to the test element (3).

3. Sterilizer according to one of claims 1 or 2, **characterized in that** the test element (3) is embodied as a preferably cylindrical capsule with an interior (8).

4. Sterilizer according to claims 2 and 3, **characterized in that** the interior (8) of the capsule is directly or indirectly in fluid communication with the sterilization chamber (1) via the probe (7).

5. Sterilizer according to claim 4, **characterized in that** the sensor (4) and the probe (7) are attached or arranged at different ends of the capsule, in particular on the frontal side.

6. Sterilizer according to any one of claims 1 to 5, **characterized in that** the sensor (4) is suitable for measuring a parameter selected from a list consisting of temperature, pressure and humidity.

7. Sterilizer according to one of claims 1 to 6, **characterized in that** the sterilization chamber (1) is subdivided into a loading area (9) and a testing area (10), the testing area (10) preferably being arranged below or to the side of the loading area (9), and the test device (2) being arranged in the testing area (10).

8. Sterilizer according to one of claims 1 to 7, **characterized in that** the cooling means (5) for cooling the test element (3) comprises a heat transfer section (11) for passing through a coolant or a refrigerant, in particular a cooling jacket or a cooling coil.

9. Sterilizer according to claim 8, **characterized in that** the cooling means (5) for cooling the test element (3) additionally comprises a supply line (12) for supplying the coolant or the refrigerant into the sterilization chamber (1) and a drain line (13) for draining the coolant or the refrigerant out of the sterilization chamber (1).

10. Sterilizer according to claim 8, **characterized in that** a supply line (12) for supplying the coolant or the refrigerant into the sterilization chamber (1) and a drain line (13) for draining the coolant or the refrigerant out of the sterilization chamber (1) are attached to the cooling means (5) for cooling the test element (3).

11. Sterilizer according to one of claims 9 or 10, **characterized in that** the coolant or the refrigerant can be circulated in a circuit.

12. Sterilizer according to one of claims 1 to 13, **characterized in that** a signal, preferably an electrical signal, generated by the sensor (4) can be passed out of the sterilization chamber (1) via a cable connection (14).

13. Sterilizer according to one of claims 1 to 13, **characterized in that** a signal, preferably an electrical signal, generated by the sensor (4) can be passed out of the sterilization chamber (1) via a radio connection comprising a transmitter and a receiver.

14. Sterilizer according to one of claims 1 to 13, **characterized in that** a signal, preferably an electrical signal, generated by the sensor (4) can be passed out of the sterilization chamber (1) inductively, in particular by means of two inductively coupled coils.

15. Sterilizer according to one of claims 1 to 14, **characterized in that** the test device (2) is at least partially inserted into a connector, in particular a validation connector (21), attached to the sterilization chamber (1).

16. Sterilizer according to claim 15, **characterized in that** the probe (7) projects out of the connector into a testing area (10) within the sterilization chamber (1).

17. Sterilizer according to one of claims 15 or 16, **characterized in that** the test device (2) is held in a stable position in the connector, and thus in the sterilization chamber, (1) via a cover plate (15) that closes off the connector.

18. Sterilizer according to claim 17, **characterized in that** the cover plate (15) is attachable to the connector via a quick-release fastener, the quick-release fastener preferably being a flange clamp (24) acting on a flange (22) that terminates the connector and on the cover plate (15).

## Revendications

1. Stérilisateur avec une chambre de stérilisation (1) et avec un dispositif de test (2) pour tester l'efficacité d'un processus de stérilisation, le dispositif de test (2) comprenant un élément de test (3) avec un capteur (4) pour mesurer au moins un paramètre et un moyen de refroidissement (5) pour refroidir l'élément de test (3), **caractérisé en ce que** l'ensemble du dispositif de test (2) avec l'élément de test (3) et le capteur (4) de l'élément de test (3) et le moyen de refroidissement (5) est disposé entièrement à l'intérieur (6) de la chambre de stérilisation (1).

2. Stérilisateur selon la revendication 1, **caractérisé en ce que** le dispositif de test (2) comprend en outre une sonde (7) qui est attachée à l'élément de test (3).

3. Stérilisateur selon l'une des revendications 1 ou 2, **caractérisé en ce que** l'élément de test (3) se présente sous la forme d'une capsule de préférence cylindrique avec un intérieur (8).

4. Stérilisateur selon les revendications 2 et 3, **caractérisé en ce que** l'intérieur (8) de la capsule est directement ou indirectement en communication fluide avec la chambre de stérilisation (1) par l'intermédiaire de la sonde (7).

5. Stérilisateur selon la revendication 4, **caractérisé en ce que** le capteur (4) et la sonde (7) sont attachés ou arrangés à des extrémités différentes de la capsule, en particulier sur le côté frontal.

6. Stérilisateur selon l'une quelconque des revendications 1 à 5, **caractérisé en ce que** le capteur (4) est apte à mesurer un paramètre choisi dans une liste constituée par la température, la pression et l'humidité.

7. Stérilisateur selon l'une des revendications 1 à 6, **caractérisé en ce que** la chambre de stérilisation (1) est subdivisée en une zone de chargement (9) et une zone de test (10), la zone de test (10) étant de préférence disposée en dessous ou sur le côté de la zone de chargement (9), et le dispositif de test (2) étant disposé dans la zone de test (10).

8. Stérilisateur selon l'une des revendications 1 à 7, **caractérisé en ce que** les moyens de refroidissement (5) de l'élément de test (3) comprennent une section de transfert thermique (11) pour le passage d'un fluide de refroidissement ou d'un réfrigérant, en particulier une chemise de refroidissement ou un serpentin de refroidissement.

9. Stérilisateur selon la revendication 8, **caractérisé en ce que** les moyens de refroidissement (5) pour refroidir l'élément de test (3) comprennent en outre une ligne d'approvisionnement (12) pour amener le fluide de refroidissement ou le réfrigérant dans la chambre de stérilisation (1) et une conduite de vidange (13) pour évacuer le fluide de refroidissement ou le réfrigérant hors de la chambre de stérilisation (1).

10. Stérilisateur selon la revendication 8, **caractérisé en ce qu'**une ligne d'approvisionnement (12) pour amener le fluide de refroidissement ou le réfrigérant dans la chambre de stérilisation (1) et une conduite de vidange (13) pour évacuer le fluide de refroidissement ou le réfrigérant hors de la chambre de stérilisation (1) sont attachées aux moyens de refroidissement (5) pour refroidir l'élément de test (3).

11. Stérilisateur selon l'une des revendications 9 ou 10, **caractérisé en ce que** le fluide de refroidissement ou le réfrigérant peut être circulé dans un circuit.

12. Stérilisateur selon l'une des revendications 1 à 13, **caractérisé en ce qu'**un signal, de préférence un signal électrique, généré par le capteur (4) peut être transmis hors de la chambre de stérilisation (1) par une connexion filaire (14).

13. Stérilisateur selon l'une des revendications 1 à 13, **caractérisé en ce qu'**un signal, de préférence un signal électrique, généré par le capteur (4) peut être transmis hors de la chambre de stérilisation (1) via une connexion radio comprenant un transmetteur et un récepteur.

14. Stérilisateur selon l'une des revendications 1 à 13, **caractérisé en ce qu'**un signal, de préférence un signal électrique, généré par le capteur (4) peut être transmis hors de la chambre de stérilisation (1) par induction, notamment au moyen de deux bobines couplées par induction.

15. Stérilisateur selon l'une des revendications 1 à 14, **caractérisé en ce que** le dispositif de test (2) est au moins partiellement inséré dans un connecteur, notamment un connecteur de validation (21), fixé à la chambre de stérilisation (1).

16. Stérilisateur selon la revendication 15, **caractérisé par le fait que** la sonde (7) sort du connecteur dans une zone de test (10) à l'intérieur de la chambre de stérilisation (1).

17. Stérilisateur selon l'une des revendications 15 ou 16, **caractérisé par le fait que** le dispositif de test (2) est maintenu dans une position stable dans le connecteur, et donc dans la chambre de stérilisation (1), par l'intermédiaire d'une plaque de recouvrement (15) qui ferme le connecteur.

18. Stérilisateur selon la revendication 17, **caractérisé en ce que** la plaque de recouvrement (15) peut être fixée au connecteur par l'intermédiaire d'une fermeture rapide, la fermeture rapide étant de préférence un collier de serrage (24) agissant sur une bride (22) qui termine le connecteur et sur la plaque de recouvrement (15).
